# EUROPEAN PATENT APPLICATION

(11) **EP 3 974 034 A1**
(43) Date of publication of application: **30.03.2022**
(21) Application number: 21198624.5
(22) Date of filing: 23.09.2021
(51) Int. Cl.: A62C 35/11, A61L 9/14, A62C 35/62, A62C 35/68, A62C 37/44

(54) **COMBINED SUPPRESSION AND STERILIZATION**

(30) Priority: 23.09.2020 US 202062706996 P
(71) Applicant: Carrier Fire & Security EMEA BV, 1831 Diegem (BE)
(72) Inventor: MOIX OLIVE, Pere, 08950 Esplugues de Llobregat (ES)
(74) Representative: Dehns

(57) **Abstract**

A system for distributing a suppression agent and a sterilization agent 111 over an area, and a method for operating the distribution system are provided. The system includes a sterilization tank 110, a suppression tank 120, and a pipe network 130. The sterilization tank 110 is used for holding the sterilization agent. The suppression tank 120 is used for holding the suppression agent. The sterilization tank 110 is operatively connected to a first valve 112. The suppression tank 120 is operatively connected to a second valve 122. The pipe network 130 is connected to the first valve 112 and the second valve 122. The pipe network 130 includes at least one spray head 131. The system may further include a control panel operatively connected to at least one of the first valve 112 and the second valve 122. The control panel, when included, may be configured to switch at least one of the first valve 112 and the second valve 122 between the open position and the closed position.

## Description

The invention relates to a combined suppression and sterilization platform. More particularly, the invention relates to the use of a shared piping network to distribute suppression agent and sterilization agent to an environment.

Environments that include surfaces that may be contacted by multiple people over a short period of time may be associated with increased health risks (e.g., from pathogen spreading). For example, one potential transmission route for bacteria, fungus, or viruses (e.g., such as SARS-CoV-2, commonly referred to as coronavirus) is the contact with fomites. These fomites may be in virtually any type of environment such as hospitals, restaurants, public transportation (e.g., passenger trains, etc.), hotels, and cruise ships. Disinfection of these environments can be tedious and time consuming. Most often these environments are cleaned manually, which may be inconsistent and ineffective if not completed thoroughly. For example, traditionally passenger trains are cleaned using disinfectant wipes to wipe down commonly touched surfaces such as handrails, doorknobs, and handles; however, if the person cleaning the environment inadvertently misses a surface or is unable to reach a surface there is potential that the pathogen may spread through the unsanitized surface.

Another risk associated with many of the above-listed environments is fire. Each of these environments (e.g., hospitals, restaurants, passenger trains, hotels, cruise ships, etc.) commonly include some sort of fire suppression system to mitigate the occurrence of a fire. Depending on the environment, these fire suppression systems often take the form of a sprinkler system, which commonly includes a pressurized tank (storing suppression agent such as water) and a piping network (made up of multiple pipes and sprinkler heads spaced above the environment so as to distribute the suppression agent from above). Often these sprinkler heads are held in a closed state by either a heat-sensitive glass bulb or a two-part metal link held together with a fusible alloy. Both the glass bulb and the metal link are designed to break at a designed temperature (e.g., caused by the fire), which, when broken, allows the fire suppression agent to exit the sprinkler head and suffocate the fire. One downside of using these types of closed state sprinklers is the inability to repeatedly use the piping network (e.g., without being serviced between each use). For example, the heat-sensitive bulbs and/or metal links may have to be replaced after each discharge. In addition, depending on the type of system (e.g., whether a dry sprinkler system or a wet sprinkler system) the piping may be full of fire suppression agent and therefore unable to be used for anything other than fire suppression. Considering that many spaces include both potential risks for pathogen spreading and fire, it may be advantageous, in certain instances, to combine both fire suppression and sterilization in a single system.

Accordingly, there remains a need for a system that combines fire suppression and sterilization in a way in which both risks can be addressed in a consistent and effective manner.

According to a first aspect a system for distributing a suppression agent and a sterilization agent over an area is provided. The system includes a sterilization tank, a suppression tank, and a pipe network. The sterilization tank is used for holding the sterilization agent. The sterilization tank is operatively connected to a first valve. The first valve has an open position and a closed position. The suppression tank is used for holding the suppression agent. The suppression tank is operatively connected to a second valve. The second valve has an open position and a closed position. The pipe network is connected to the first valve and the second valve. The pipe network includes at least one spray head.

Optionally, the system further includes a control panel operatively connected to at least one of the first valve and the second valve, the control panel configured to switch at least one of the first valve and the second valve between the open position and the closed position.

Optionally, the system further includes a fire sensor operatively connected with the control panel, the control panel configured to switch the second valve to the open position when the fire sensor detects a fire in the area.

Optionally, the fire sensor includes at least one of a heat sensor and an optical sensor.

Optionally, the system further includes a manually activated switch connected with the Optionally, the distribution system further includes a control panel, the control panel operatively connected to at least one of the first valve and the second valve, the control panel configured to switch at least one of the first valve and the second valve between the open position and the closed position.

Optionally, the distribution system includes a fire sensor, the fire sensor configured to detect when at least a portion of the area requires suppression.

Optionally, the fire sensor includes at least one of a heat sensor and an optical sensor.

Optionally, the pipe network includes unpressurized dry piping.

Optionally, the sterilization agent is in a substantially gaseous phase when released from the sterilization tank, and the suppression agent is in a substantially liquid phase when released from the suppression tank.

Optionally, the sterilization agent includes at least one of: ozone and chlorine dioxide.

Optionally, the suppression agent includes at least one of: water and a foam solution.

The subject matter, which is regarded as the disclosure, is particularly pointed out and distinctly claimed in the claims at the conclusion of the specification. The following descriptions of certain example embodiments, with reference to the drawings should not be considered limiting in any way. In the accompanying drawings, like elements are numbered alike:
FIG. 1 is a schematic illustration of a system for distributing a suppression agent and a sterilization agent over an area, the system including a pipe network with at least one spray head.
FIG. 2 is a schematic illustration of an exemplary pipe network for the distribution system shown in FIG. 1.
FIG. 3 is a flow diagram illustrating a method of operating a distribution system including a sterilization tank, a suppression tank, and a pipe network.

Fire suppression and sterilization are typically configured separately. Sterilization is often a manual process, completed either by manually spraying an aerosol disinfectant or manually wiping a disinfectant wipe. Fire suppression on the other hand is typically an automated process, that includes at least one fire detector (to detect the fire), a control panel (to receive and process the detection signal from the fire detector), a suppression tank (to store the fire suppression agent), a valve (to release the fire suppression agent), a pipe network (to guide the flow of the fire suppression agent), and a sprinkler or spray head (to disperse the fire suppression agent over the fire). It should be understood that a sprinkler and a spray head are functionally and structurally different. Sprinklers are closed state devices and spray heads are open state devices. Sprinklers are typically used in systems where the thermal activation of the sprinkler activates the discharge. This thermal activation can either be the bursting of a glass bulb or disconnect of a metal link when exposed to a certain temperature (e.g., typically between 57 to 141°C), which allows high pressure fire suppression agent (e.g., commonly water) to flow through the nozzles. Spray heads, as mentioned above, are always open and do not include glass bulbs or metal link for activation. One benefit of using spray heads over sprinklers, is that the spray heads can be repeatedly used without servicing. As described below, the distribution system described herein includes at least one spray head (e.g., instead of a sprinkler).

The design and configuration of the distribution system described herein enables fire suppression and sterilization to be combined into a single platform (e.g., using the same or substantially the same pipe network). For example, sterilization agent and suppression agent may flow through the same piping when respectively disbursed. It should be appreciated that sterilization agent and fire suppression agent may not be disbursed at the same time. It is envisioned that both fire suppression and sterilization may be completed in an automated manner with the distribution system. For example, the distribution system may remove the need to manually spray aerosol disinfectant or manually wipe disinfectant wipes, which, by removing potential human error, may increase the consistency and effectiveness of the sterilization process. Although described herein to be effective at reducing the risk associated with pathogen spreading, the distribution system described herein may also be effective at exterminating insects and/or pests (e.g., mice, etc.). It should be appreciated that the sterilization agent may vary depending on the desired removal.

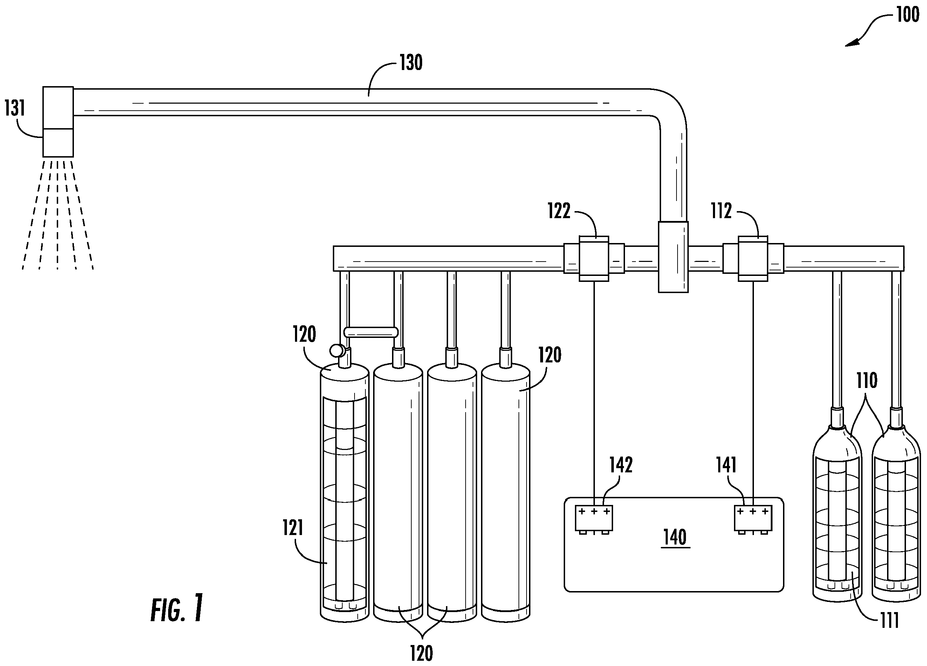

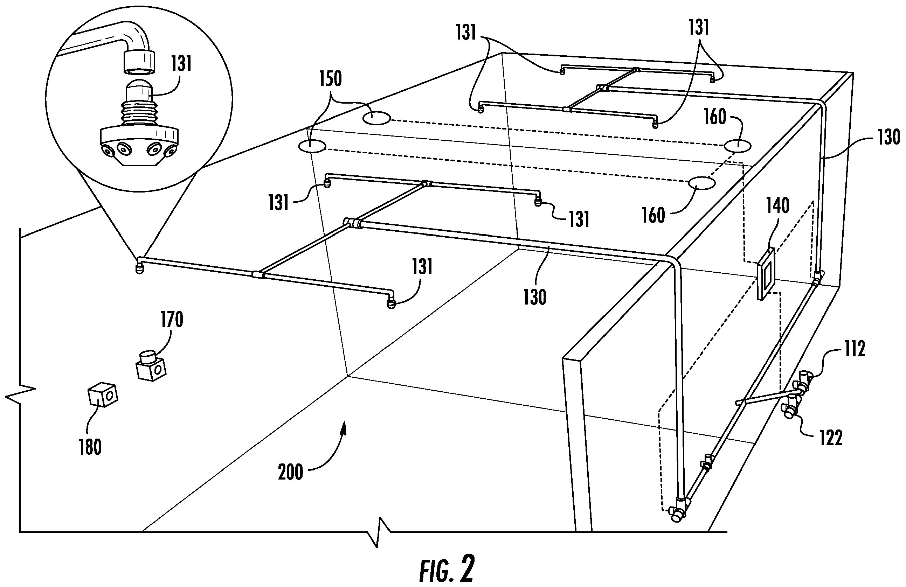

With reference now to the Figures, a schematic illustration of an exemplary system 100 for distributing a suppression agent 121 and a sterilization agent 111 over an area 200 is shown in FIG. 1. It should be appreciated that the system 100 may be referred to herein as a distribution system 100. As shown in FIG. 1, the distribution system 100 may include a sterilization tank 110, a suppression tank 120, and a pipe network 130. The sterilization tank 110 is used for holding the sterilization agent 111. The suppression tank 120 is used for holding the suppression agent 121. As shown in FIG. 1, the distribution system 100 may include multiple sterilization tanks 110 and suppression tanks 120. The number of sterilization tanks 110 and suppression tanks 120 may vary depending on the size of the area 200 (shown in FIG. 2). For example, more sterilization tanks 110 and/or suppression tanks 120 may be needed for larger areas 200. The sterilization tank(s) 110 are operatively connected to a first valve 112. The first valve 112 has an open position (allowing the release of the sterilization agent 111) and a closed position (preventing the release of the sterilization agent 111). The suppression tank(s) 120 are operatively connected to a second valve 122. The second valve 122 has an open position (allowing the release of the suppression agent 121) and a closed position (preventing the release of the suppression agent 121). The distribution system 100 includes a pipe network 130 connected to the first valve 112 and the second valve 122. The pipe network 130 includes at least one spray head 131 (e.g., to disburse agent(s) 111, 121 within the area 200). It should be appreciated that the pipe network 130 may include any number of pipes and any number of spray heads 131. To ensure that the pipe network 130 remains unblocked, the pipe network 130 may be made of unpressurized dry piping. Unpressurized dry piping may be interpreted to mean that the pipe network 130 is not filled with either agent (e.g., suppression agent 121 or sterilization agent 111). For example, the pipe network 130 may be substantially clear until a valve 112, 122 is opened. This may allow the distribution system 100 to be flexible and be able to disburse either agent 111, 121 at any given time.

The distribution system 100 described herein may be designed for repeated use (e.g., without having to service the spray head(s) 131). For example, the distribution system 100 may be able to complete multiple distributions (e.g., more than one distribution of sterilization agent 111 and/or suppression agent 121) without servicing the spray head(s) 131. The distribution of sterilization agent 111 and/or suppression agent 121 may be dependent on the opening of valves (e.g., the first valve 122 for suppression agent and the second valve 112 for the sterilization agent 111). It is envisioned that at least one of the valves 112, 122 may be solenoid valves (e.g., capable of being remotely controllable). The opening/closing of these valves 112, 122 may be completed by a control panel 140. For example, the control panel 140, when included, may be connected (e.g., using one or more wired or wireless connection) to at least one of the first valve 112 and the second valve 122. The control panel 140 may be configured to switch at least one of the first valve 112 and the second valve 122 between the open position and the closed position (e.g., by transmitting a signal to the respective valve 112, 122). It should be appreciated that signal from control panel 140 to the respective valve 112, 122 may be transmitted wirelessly (e.g., using a short-range communication such as Bluetooth, Bluetooth Low Energy (BTLE), Zigbee, infrared, ultra-wide band (UWB), and Wi-Fi) or over a wired communication (e.g. UART, Serial, Fiber-optic, SPI or Ethernet cable).

The determination of whether or not to open the second valve 122 may be dependent on whether or not a fire is detected in the area 200. For example, as shown in FIG. 2, the control panel 140 may be connected (e.g., using one or more wired or wireless connection) with a fire sensor 150 so as to open the second valve 122 when a fire is detected in the area 200. The fire sensor 150 may include at least one of a heat sensor (e.g., operating using infrared technology) and an optical sensor (e.g., operating using light reflection principles) to detect the presence of a fire within the area 200. For example, the optical sensor may include one or more emitter(s) and one or more receiver(s) to allow the fire sensor 150 to detect smoke, which may indicate the presence of a fire in the area 200. It is envisioned that the fire sensor 150 may use at least one of a multi-wave, multi-angle detection method, a backscatter detection method, and a forward scatter detection method to detect the presence of fire in the area 200. Regardless of the way in which the fire sensor 150 detects the presence of fire in the area 200, the fire sensor 150 may be configured to internally process the readings of the emitter(s) and/or receiver(s), or transmit (e.g., using one or more wired or wireless connections) the readings to the control panel 140 for processing. It should be appreciated that the control panel 140 and/or the fire sensor 150 may include a processor (e.g., a microprocessor) to process the readings of the emitter(s) and/or receiver(s). Regardless of where the processor is located, the respective processor may be, but is not limited to, a single-processor or multi-processor system of any of a wide array of possible architectures, including field programmable gate array (FPGA), a central processing unit (CPU), application specific integrated circuits (ASIC), digital signal processor (DSP) or graphics processing unit (GPU) hardware arranged homogenously or heterogeneously. It is envisioned that the determination of a fire (e.g., by the fire sensor 150 or the control panel 140) may trigger an alarm 180, which may be located in the area 200. The alarm 180 may generate at least one of an audible signal and a visual indicator (e.g., an illumination of a light).

It should be appreciated that the first valve 112 and/or the second valve 122 may be manually activated (e.g., using a switch 142, 141, 180 operatively connected with the control panel 140). As shown in FIG. 1 the switch 141, 141 may be located on the control panel 140. However, it is envisioned, as shown in FIG. 2, that a switch 180 may be located remotely from the control panel 140. Regardless of where the switch 141, 142, 180 is located, an operator of the distribution system 100 may be able to manually flip a switch (e.g., the switch 141) to position a valve (e.g., the first valve 112) in the open position (e.g., to release the sterilization agent 111). Although described as a switch 141, 142, 180 it should be appreciated that any actuation mechanism may be utilized. For example, the switch 141, 142, 180 may be a depressible button (not shown) in certain instances.

Depending on the type of sterilization agent 111 utilized, it may be advantageous (e.g., for safety and/or health reasons) to include a presence detection sensor 160 to ensure that sterilization agent 111 is not released when a person is present in the area 200. As shown in FIG. 2, the presence detection sensor 160 may be operatively connected with the control panel 140 (e.g., using one or more wired or wireless connection). The presence detection sensor 160 may be configured to prevent the first valve 112 from switching to the open position when the presence detection sensor 160 detects a person in the area 200. This may help prevent the person from being exposed to the sterilization agent 111, which may prevent the person from having any associated health risks from the sterilization agent 111. It should be appreciated that health risks may vary depending on the type of sterilization agent 111 utilized.

To detect the presence of a person in the area 200, the presence detection sensor 160 may utilize at least one of a radar sensor (e.g., detecting movement, heartbeat and/or respiration using radio waves) and an infrared sensor (e.g., detecting heat). Regardless of the way in which the fire sensor 150 detects the presence of a person in the area 200, the presence detection sensor 160 may be configured to internally process the readings, or transmit (e.g., using one or more wired or wireless connections) the readings to the control panel 140 for processing. It should be appreciated that the control panel 140 and/or the presence detection sensor 160 may include a processor (e.g., a microprocessor) to process the readings. Regardless of where the processor is located, the respective processor may be, but is not limited to, a single-processor or multi-processor system of any of a wide array of possible architectures, including field programmable gate array (FPGA), a central processing unit (CPU), application specific integrated circuits (ASIC), digital signal processor (DSP) or graphics processing unit (GPU) hardware arranged homogenously or heterogeneously.

The phase in which the suppression agent 121 and sterilization agent 111 are in when released may have a direct effect on their ability to perform their associated task. For example, the sterilization agent 111 may be in a substantially gaseous phase when released from the sterilization tank 110, which, as described below, may help ensure all surfaces are sterilized. A substantially gaseous phase may mean that a greater proportion (e.g., percentage) of the sterilization agent 111 may be in a gas or vapor phase than liquid phase when released from the sterilization tank 110. Conversely, the suppression agent 121 may be in a substantially liquid phase when released from the suppression tank 120. A substantially liquid phase may mean that a greater proportion (e.g., percentage) of the suppression agent 121 may be in a liquid (e.g., in the form of a foam or mist) phase than gas or vapor phase when released from the suppression tank 120. By being in a substantially liquid phase, the suppression agent 121 may be able to create a stronger barrier between the fuel source and the oxygen source (e.g., compared to if the suppression agent 121 was in a substantially gaseous phase). By being in a substantially gas or vapor phase, the sterilization agent 111 may be able to fill the entire volume of the area 200 instead of simply covering the upper surfaces of objects within the area 200. Filling the volume of the area 200 may allow the sterilization agent 111 to come into contact with all surfaces (e.g., including the non-upward facing surfaces and the areas which are not accessible by hand), thereby providing more thorough sterilization than would be possible through manual cleaning.

As mentioned above, it is envisioned that the design and configuration of the distribution system 100 described herein may make it possible to complete both effective fire suppression (e.g., using suppression agent 121) and sterilization (e.g., using a sterilization agent 111) in a single platform (e.g., through a shared pipe network 130). It should be appreciated that although any fire suppression agent 121 may be utilized to complete the fire suppression, the fire suppression agent 121 may be water or a foam solution in certain instances. Additionally, depending on the type of sterilization being completed, the sterilization agent 111 may vary. For example, if sterilizing to prevent a pathogen (e.g., such as coronavirus) from spreading the sterilization agent 111 may be ozone (O3) in certain instances. If sterilizing to exterminate insects and/or pests, the sterilization agent 111 may be chlorine dioxide (ClO2) in certain instances. For each of these sterilization agents 111, the sterilization agent 111 may be in a substantially gaseous phase when released from the sterilization tank 110, which may help to ensure that thorough sterilization is completed.

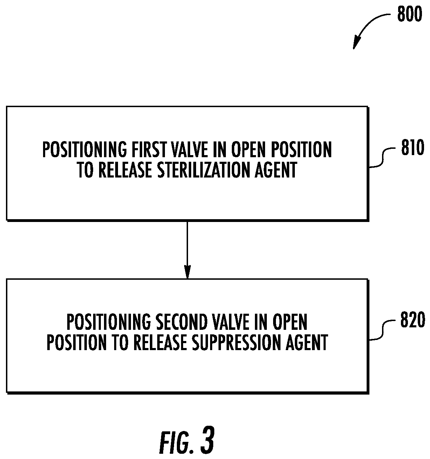

The distribution system 100 described herein may be useful in a variety of different environments (e.g., hospitals, restaurants, passenger trains, hotels, cruise ships, etc.). For example, the distribution system 100 may be useful in any type of environment where both fire suppression and sterilization may be needed. An exemplary method 800 of operating a distribution system 100 is illustrated in FIG. 3. The method 800 may be performed, for example, using the exemplary distribution system 100 shown in FIG. 1, which includes a sterilization tank 110 operatively connected to a first valve 112, a suppression tank 120 operatively connected to a second valve 122, and a pipe network 130 connected to the first valve 112 and the second valve 122. The first valve 112 and the second valve 122 each have an open position (allowing the release of agent 111, 121, respectively) and a closed position (preventing the release of agent 111, 122, respectively). The method 800 includes step 810 for positioning the first valve 112 in an open position (defined by the release of sterilization agent 111 to an area 200) when at least a portion of the area 200 requires sterilization. The determination of whether an area 200 requires sterilization may be automatic (e.g., through detecting the presence of a pathogen or pest, or based upon a certain time). For example, the sterilization may be completed periodically (e.g., at certain time intervals, such as every hour or every two hours of a certain day). However, it should be appreciated that the determination of whether an area 200 requires sterilization may be manual (e.g., determined by a person). The method 800 includes step 820 for positioning the second valve 122 in an open position (defined by the release of suppression agent 121 to an area 200) when at least a portion of the area 200 requires suppression. The determination of whether an area 200 requires suppression may be automatic (e.g., through detecting the presence of a fire with a fire sensor 150). However, it should be appreciated that the determination of whether an area 200 requires suppression may be manual (e.g., determined by a person).

The use of the terms "a" and "and" and "the" and similar referents, in the context of describing the invention, are to be construed to cover both the singular and the plural, unless otherwise indicated herein or cleared contradicted by context. The use of any and all example, or exemplary language (e.g., "such as", "e.g.", "for example", etc.) provided herein is intended merely to better illuminate the invention and does not pose a limitation on the scope of the invention unless otherwise claimed. No language in the specification should be construed as indicating any non-claimed elements as essential to the practice of the invention.

While the present disclosure has been described with reference to an exemplary embodiment or embodiments, it will be understood by those skilled in the art that various changes may be made and equivalents may be substituted for elements thereof without departing from the scope of the present invention, which is defined by the claims. In addition, many modifications may be made to adapt a particular situation or material to the teachings of the present disclosure. Therefore, it is intended that the present invention not be limited to the particular embodiment disclosed as the best mode contemplated for carrying out this present disclosure, but that the present invention will include all embodiments falling within the scope of the claims.

## Claims

1. A system for distributing a suppression agent and a sterilization agent over an area, the system comprising:
a sterilization tank for holding the sterilization agent, the sterilization tank operatively connected to a first valve, the first valve comprising an open position and a closed position;
a suppression tank for holding the suppression agent, the suppression tank operatively connected to a second valve, the second valve comprising an open position and a closed position;
a pipe network connected to the first valve and the second valve, the pipe network comprising at least one spray head.

2. The system of claim 1, comprising a control panel operatively connected to at least one of the first valve and the second valve, the control panel configured to switch at least one of the first valve and the second valve between the open position and the closed position.

3. The system of claim 2, comprising a fire sensor operatively connected with the control panel, the control panel configured to switch the second valve to the open position when the fire sensor detects a fire in the area.

4. The system of claim 3, wherein the fire sensor comprises at least one of a heat sensor and an optical sensor.

5. The system of claim 2, further comprising a manually activated switch connected with the control panel, the manually activated switch configured to switch the first valve between the open position and the closed position.

6. The system of claim 2, further comprising a presence detection sensor operatively connected with the control panel, the presence detection sensor configured to prevent the switching of the first valve to the open position when the presence detection sensor detects a person in the area.

7. The system of claim 6, wherein the presence detection sensor comprises at least one of a radar sensor and an infrared sensor.

8. The system of claim 1, wherein the sterilization agent comprises a substantially gaseous phase when released from the sterilization tank.

9. The system of claim 1, wherein the suppression agent comprises a substantially liquid phase when released from the suppression tank.

10. The system of claim 1, wherein the pipe network comprises unpressurized dry piping.

11. The system of claim 1, wherein the sterilization agent comprises at least one of: ozone and chlorine dioxide.

12. The system of claim 1, wherein the suppression agent comprises at least one of: water and a foam solution.

13. A method for operating a distribution system, the distribution system comprising a sterilization tank operatively connected to a first valve, a suppression tank operatively connected to a second valve, and a pipe network connected to the first valve and the second valve, the first valve and the second valve each respectively comprising an open position and a closed position, the method comprising:
positioning the first valve in an open position, the open position of the first valve being defined by the release of a sterilization agent to an area, the first valve being placed in the open position when at least a portion of the area requires sterilization; and
positioning the second valve in an open position, the open position of the second valve being defined by the release of a suppression agent to the area, the second valve being placed in the open position when at least a portion of the area requires suppression.

14. The method of claim 13, wherein the distribution system comprises a system as claimed in any of claims 1 to 12.
